(19) Europäisches Patentamt
European Patent Office
Office européen des brevets

(11) **EP 4 372 617 A1**

(12) **EUROPEAN PATENT APPLICATION**

(43) Date of publication:
**22.05.2024  Bulletin 2024/21**

(21) Application number: **23169568.5**

(22) Date of filing: **24.04.2023**

(51) International Patent Classification (IPC):
**G06N 3/045** (2023.01)   **G06N 3/0895** (2023.01)
**A61F 2/48** (2006.01)   **A61F 2/68** (2006.01)
**G06N 3/0464** (2023.01)   **G06N 3/096** (2023.01)

(52) Cooperative Patent Classification (CPC):
**G06N 3/045; A61F 2/48; G06N 3/0464;**
**G06N 3/0895; G06N 3/096;** A61F 2/68

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Georg-August-Universität Göttingen**
**Stiftung**
**Öffentlichen Rechts, Universitätsmedizin**
**37099 Göttingen (DE)**

(72) Inventors:
• **Dey, Sharmita**
  **37075 Göttingen (DE)**
• **Schilling, Arndt**
  **37075 Göttingen (DE)**

(74) Representative: **Betten & Resch**
**Patent- und Rechtsanwälte PartGmbB**
**Maximiliansplatz 14**
**80333 München (DE)**

(54) **METHOD AND APPARATUS FOR TRAINING A COMPUTER-IMPLEMENTED MACHINE-LEARNING MODEL**

(57)    A method for training a computer-implemented machine-learning model $f$ for controlling a system by control parameter **y,** wherein said model has been trained to predict a target variable **y** = $f(x)$ based on an input feature value **X**, where **X** is an input value feature value obtained by one or more sensors and used to generate by said model said target variable **y** as control parameter to thereby control said system,
wherein said model $f$ has been trained using a first set of training data $(y_k, x_k)$ corresponding to k =1... N time points to find the trained machine learning model $f^*$
wherein a backward model $g^*$ has been trained based on at least a part of said first set of training data $(y_k, x_k)$ to predict an input feature value $x_{k+1}$ based on its preceding input feature value $x_k$ and the corresponding target variable $y_k$, said method comprising:
using said backward model $g^*$ to generate second set of training data, which comprises the future input feature values $x_{k+1}$ predicted by the backward machine learning model $g^*$ and the corresponding ground truth variable $y_{k+1}$,
training said model f, which has been trained using said first set of training data, using said replay buffer as said second set of training data.

Fig. 1

**EP 4 372 617 A1**

**Description**

**FIELD OF THE INVENTION**

The present invention relates to a method and an apparatus for training a computer implemented machine-learning model.

**BACKGROUND OF THE INVENTION**

Machine-learning models are used in many fields, not only to analyze and categorize data, but also to control a technical apparatus, such as autonomously driving cars, machines in manufacturing plants, or also to control for example bionic limbs. The following description will mostly relate to this field of application, where a bionic limb, which has embedded one or more motors, is controlled by a machine-learning model. However, the described method can as well be applied to other application scenarios such as the control of an autonomously driving vehicle or the control of a machine in a plant. Wherever a control output is generated to control an apparatus, its actuators or motors, based on one or more input values, which have been obtained by one or more sensors, to thereby perform real-time control of the apparatus, the machine learning methods described in the following can in principle be applied.

Lower limb amputation and neuromuscular impairment hinder mobility and limit the quality of life. Bionic limbs with embedded motors may be programmed to emulate the motion of a human limb. For a bionic limb to generate human-level movement for assisting patients, its programming strategy should preferably incorporate how the natural synergy in humans evolves across varying tasks and environments.

Lower limb amputation and neuromuscular impairment impedes natural locomotion and restricts the quality of life. Physically impaired individuals rely on assistive devices such as prosthetic limbs or orthoses to restore their daily locomotion tasks. The passive prostheses such as the Energy Storage and Release (ESAR) foot and the semi-active variable damping prostheses do not provide adequate net positive work required for natural gait such as standing up from a sitting position, walking fast, running, climbing stairs or slopes. Consequently, the physically challenged individuals need to compensate for the missing support by increasing their energy expenditure, e.g., by performing exaggerated residual body movements, thus leading to unnatural gait. Therefore, individuals with a passive replacement of the missing limbjoint often exhibit unnatural gait asymmetry that leads to several problems such as increased forces acting on the sound joints, fatigue, increased oxygen consumption, and metabolic cost.

To ameliorate this deficiency, powered prostheses or orthoses (p/o) with embedded motors may be programmed to emulate the motion of a biological limb. Such a bionic limb can co-exist and co-function with the human body to replace the missing limb in the different locomotion scenarios. However, complementing the human body with a bionic limb is not a standalone phenomenon, but has to be in tandem with the motion of the entire body. For this, a programming strategy needs to learn how the biological synergistic coupling in the human body functions and evolves across varying motion conditions.

Several methods have been proposed to approximate this biological synergy, the most traditional and popular being the finite-state-machines, where a finite stateaction space is typically modelled to simplify the biological states (i.e., kinematics, kinetics, or the muscular activation of the residual body of the amputated individual) during gait. A tabular value representation is maintained based on the finite states to operate the bionic limb. The different state transitions are governed by switching rules that make decisions based on the biological state. To support finer phases of gait (e.g., initial stance, mid-stance, early swing) or multiple locomotion tasks (e.g., level-ground walking, walking on inclined ground, stair ambulation), the number of states to be defined, parameters to be tuned, tabular representations to be maintained, and, switching rules to be defined increases, leading to an explosion of the parameter space.

A more intuitive way to imitate the biological synergistic coupling would be to learn it from able-bodied human demonstrations instead of explicit coding, and apply this synergy-approximated model to predict the missing limb motion for amputees. This approach not only models the smooth nature of gait more accurately but also models the natural transitions across locomotion tasks, speeds and gait cycles without having to maintain hard coded rules and transition heuristics. The learned gait models could be personalized by conserving the search space for human demonstrations within similar anthropomorphic features as that of the physically impaired subject.

An important factor missed by these studies is that when applying a learned model for predicting the movement signals for the physically challenged individuals, the prediction errors might affect the gait patterns causing perturbation to the input patterns. In such cases, a non-adaptive model might become unusable when errors accumulate over time. Further, temporal changes in gait patterns caused by other exteroceptive and interoceptive factors such as terrain, speed, fatigue and new locomotion tasks motivates the need for an adaptive gait model that need not be retrained completely.

It is therefore desirable to provide a training method for a machine-learning method, which is more resilient against prediction errors, which may accumulate over time. It is further desirable that the training method for a machine-learning model enables the model to adapt to new tasks such as new scenarios, in which the model is applied.

**SUMMARY OF THE INVENTION**

According to one aspect there is provided a method training a computer-implemented machine-learning model $f$ for controlling a system by control parameter **y,** wherein said model has been trained to predict a target variable **y** = $f(x)$ based on an input feature value **X,** where **X** is an input value feature value obtained by one or more sensors and used to generate by said model said target variable **y** as control parameter to thereby control said system,

wherein said model $f$ has been trained using a first set of training data ($\mathbf{y}_k$, $\mathbf{x}_k$) corresponding to k =1... N time points to find the trained machine learning model $f^*$
wherein a backward model g* has been trained based on at least a part of said first set of training data ($\mathbf{y}_k$, $\mathbf{x}_k$) to predict an input feature value $\hat{\boldsymbol{x}}_{k+1}$ based on its preceding input feature value $\mathbf{x}_k$ and the corresponding target variable $\mathbf{y}_k$, said method comprising:

using said backward model g* to generate second set of training data, which comprises the future input feature values $\hat{\boldsymbol{x}}_{k+1}$ predicted by the backward machine learning model g* and the corresponding ground truth variable $\mathbf{y}_{k+1}$,
training said model f, which has been trained using said first set of training data, using said replay buffer as said second set of training data.

The generation of a backward model together with the generation of a second set of training data as some kind of "replay buffer" based on the backward model enables the introduction of an error aware replay buffer, which improves the model f by making it more resilient against errors, especially prediction errors, or against changes in the environment.

According to a further aspect said second set of training data comprises pairs of values comprising a future input feature value $\hat{\boldsymbol{x}}_{k+1}$ , which is predicted by the trained backward model g* as g*($\boldsymbol{x}_k$, $f(\boldsymbol{x}_k)$) with $\boldsymbol{x}_k$ being a ground truth input feature value from the first set of training data and $f(\boldsymbol{x}_k)$ being its corresponding predicted target variable predicted by model f, and with $\mathbf{y}_{k+1}$ being the ground truth value from the first set of training data corresponding to the predicted input feature value $\hat{\boldsymbol{x}}_{k+1}$.

By generating the backward model in this manner there is introduced or trained a prediction of the input feature value rather than - as usually done - a prediction of the output value. This introduces some new notion into the prediction, namely the attempt to predict the "input value", i.e. the "control variable" rather than the "output variable" or the "controlled variable". This tries to "infer" some "behavior" of the input data of the control loop based on the ground truth output data and the preceding pair of input and output, which in some sense "inverts" the normal prediction and control direction. In this manner, it enables to introduce some kind of "error" into the training mechanism, in particular by using the backward model to generate a second set of training data to be used for further training.

According to a further aspect the model g* is trained using said first set of training data or a subset thereof, the first set of training data comprising ($\mathbf{y}_k$, $\mathbf{x}_k$) for k=1... N, with N being the number of samples of the first set of training data.

In this manner the first set of training data, partly or as a whole, is used to train also the backward model, which then is used for generating the second set of training data.

According to a further aspect the pairs of values of the second set of training data correspond to the ground truth variable $\mathbf{y}_{k+1}$ of the first set of training data and the value $\hat{\boldsymbol{x}}_{k+1}$ predicted by the backward model g* as as g*($\boldsymbol{x}_k$, $f(\boldsymbol{x}_k)$) for all k = 1 to M-1, where M is the number of samples or a subset of samples of a separate set of generative data ($\mathbf{y}_k$, $\mathbf{x}_k$) comprising ground truth values, which have not been used for training the models f or g*.

Using the separate set of generative data ($\mathbf{y}_k$, $\mathbf{x}_k$) having M-1 samples, which also are ground truth values like the first set of training data, enables the generation of the second set of training data for further training the model f.

According to a further aspect said method comprises further training said trained model by said second set of training data generated using said trained backward machine learning model g* which has been trained using at least a part of said training data to learn a functional mapping g* to predict a future value $\mathbf{x}_{k+1}$ based on a temporally preceding feature value $\mathbf{x}_k$ and its corresponding target value $\mathbf{y}_k$ so that $\mathbf{x}_{k+1}$ = g($\mathbf{x}_k$, $\mathbf{y}_k$) by finding the trained backward model as

$$\mathbf{g}^* = arg\ min\ \left(\textstyle\sum_{k=1}^{K-1}(\boldsymbol{x}_{k+1} - g(\boldsymbol{x}_k, \boldsymbol{y}_k))^2\right)$$

wherein said trained backward model g* is used to generate said second set of training data consisting of the future input feature values $\hat{\boldsymbol{x}}_{k+1}$ predicted by the trained backward machine learning model $g^*(\boldsymbol{x}_k, \boldsymbol{y}_k)$ and the corresponding ground truth variable $\mathbf{y}_{k+1}$ from the set of generative data.

In this manner there can be implemented a generation of the second set of training data such that it can be used to improve the model f* by further training it using the second set of training data.

According to a further aspect said model f is a neural network (**X**; Θ) , preferably a temporal convolutional network TCN, with **X** being the input feature value and Θ being the trainable parameters of the one or more layers of the neural network;

wherein

said model has been trained using a first set of training data $(y_k, x_k)$ corresponding to k =1 ... N time points to find the trained neural network having the trained parameters Θ* of the at least one layer of the neural network as

$$\Theta^* = \arg\min (\| y - f(X; \Theta \|_2)$$

wherein said second training set is used to further train said model by finding improved trained parameters of the at least one layer of the neural network as

$$\Theta^* = \arg\min (\| y - f(\hat{X}; \Theta \|_2)$$

where

$$\hat{X} = g^*(\mathbf{X}, f(\mathbf{X})).$$

In this manner there can be implemented a method for further training a model f, which has been already trained using a first data set, by a second data set, which is based on a backward model and a forward prediction, and which introduces an error aware component, thereby making the model more resilient against prediction errors and changing environments.

According to a further aspect said model f has at least two layers with corresponding training parameters, a shared layer $\Theta_s$ and one or more task specific layers $\Theta_t$ , where when a new task t' is to be learned, a new task specific layer $\Theta_{t'}$ is added and the model f is retrained to adapt to the new task t' to thereby obtain the trained model f having the layers $\Theta_s$ and $\Theta_t$ as

$$\Theta_s^*, \Theta_t^* = \underset{\{\Theta_s, \Theta_t, t \in T\}}{\arg\min} \left( \left\| \mathbf{y}_{t'} - f(\mathbf{X}_{t'}; \Theta_s, \Theta_{t'}) \right\|_2 + \sum_{t \in T} \left\| \mathbf{y}_t - f(\hat{\mathbf{X}}_t; \Theta_s, \Theta_t) \right\|_2 \right), t \in T \cup t'$$

where

$$\hat{X}t = g_t^*(\mathbf{X}_t, f(\mathbf{X}_t)).$$

In this manner, the model can be adapter to different tasks using separate task layers, which are trained using a first set of training data set and a second set of training data.

According to a further aspect said control parameter **Y** is a control value for controlling a machine or apparatus, preferably by controlling an engine, a motor, a steering mechanism, or an actuator of said machine or apparatus, and wherein said machine or apparatus preferably is a machine for manufacturing, driving, flying, or moving a physical entity, more preferably one of the following:

a car;
a plane;
a vehicle;
a robot;
a machine used in a manufacturing plant.

In this manner, the model can be applied to control some real-world machines or apparatuses in a way, which is more resilient against prediction errors of the prediction model.

According to a further aspect, said controlled machine or apparatus is a virtual machine or apparatus, which is simulated in a virtual scenario, and which is controlled by said model f.

The implementation of the controlled machine or apparatus as a simulated machine or apparatus enables the application, development, testing and improvement of the model even without experiments in the real-world but rather based on simulation scenarios. This can become more and more important, as digital twins are used for development, monitoring and implementation of real world scenarios such as machines, apparatuses, and even manufacturing plants.

According to a further aspect the input feature value $X$ is based on sensor data, wherein the sensor data preferably comprises one or more of the following:

> position sensor data;
> velocity sensor data;
> pressure sensor data;
> voltage sensor data;
> current sensor data;
> magnetic field sensor data;
> temperature sensor data;
> acceleration sensor data;
> wearable sensor data.

These are suitable implementations of sensor data, which can be used as input features for the model to generate based thereon the control parameter for controlling the system.

According to a further aspect said control parameter $Y$ is a control value for controlling one or more motors or actuators of a bionic limb, which is controlled by said model to enable a patient to emulate the motion of a human limb.

In this manner, a resilient control for a bionic limb can be implemented.

According to a further aspect, the input feature value $X$ is based on wearable sensor data.

This enables the control of the bionic limb based on sensored input data as input feature values used in the model.

According to a further aspect the control parameter $Y$ comprises one or more of an angle, a speed, an acceleration, or a force for controlling one or more actuators or motors of said bionic limb.

According to a further aspect said control parameter $Y$ is a control value for controlling one or more motors or actuators of a car, preferably one or more of a steering wheel angle, a deceleration value, or an acceleration value, and wherein the input feature value $X$ is based on sensor data, preferably one or more of camera-based sensor data, LIDAR sensor data, or radar sensor data.

In this manner, the training method can be used to provide an improved control mechanism for an autonomous vehicle.

According to a further aspect there is provided a computer-implemented machine-learning model *f* for controlling a system by control parameter **y,** wherein said model has been trained using a method according to one of the previously described aspects.

According to a further aspect there is provided the use of a computer-implemented machine-learning model *f* for controlling a system by control parameter **y,** wherein said model has been trained using a method according to one of the previously described aspects.

According to a further aspect there is provided computer program, preferably recorded on a computer redable medium, comprising program executable code, which when being executed by a computer enables said computer to perform a method according to one of the previous aspects.

## DESCRIPTION OF THE DRAWINGS

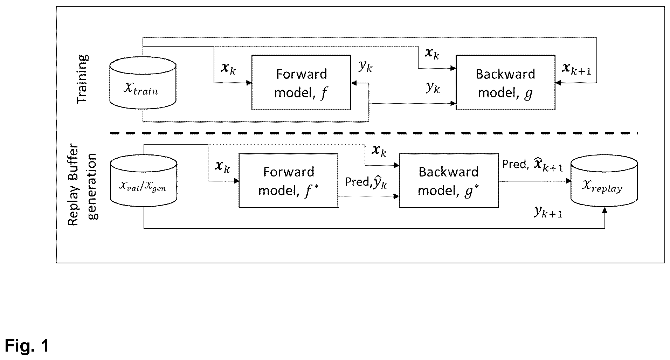

Fig. 1 illustrates a training method according to one embodiment.

## DETAILED DESCRIPTION

A first embodiment of a training method for a computer-implemented machine-learning model f will be described in connection with Fig. 1.

In the upper part of Fig. 1 there is illustrated a forward model f. This forward model f can be used to control a technical system or apparatus, e.g. by outputting a predicted variable based on an input feature value.

For that purpose, the computer-implemented machine-learning model *f* can be adapted to be used for controlling a system by control parameter **y** by training the model to predict a target variable $y = f(x)$ based on an input feature value $X$.

Here the input feature value **X** and the control variable **y** both may be vectors having multiple components. The model is trained by a first set of training data $X_{train}$, which comprises 1... N pairs of values $\mathbf{X}_k$ and $\mathbf{y}_k$ which correspond respectively to instances k of time, with k preceding k+1 in time and with a pair of values $\mathbf{X}_k$ and $\mathbf{y}_k$ at time k being ground truth values of $\mathbf{X}_k$ and $\mathbf{y}_k$.

Thereby $\mathbf{X}_k$ is an input value feature value obtained by one or more sensors, and $\mathbf{y}_k$ is the corresponding control value to control the system. For example, $\mathbf{X}_k$ can be a sensor value from an optical sensor (e.g. a distance value), an acoustic sensor (e.g. a noise volume), or an acceleration sensor, and $\mathbf{y}_k$ is e.g. an actuator control value for an actuator to be controlled, like e.g. a steering wheel, a motor speed, an angle value, or the like, which should be outputted by the model f to correctly control the system. In other embodiments other sensor values from other sensors may be used, and other control values to control the system may be generated.

Pairs of ground truth values $\mathbf{X}_k$ and $\mathbf{y}_k$ for 1 ... M from the training set $X_{train}$ as first set of training data are then used to train the model f. By training model f using this first set of training data it then becomes trained model f*.

Once the model f has been trained into model f, there is then trained a further model g. This may be called a "backward model", because it is not oriented towards a certain instance of time k regarding feature input and prediction, but in some sense it is "backward oriented", because it is trained to predict a future input feature value $\mathbf{x}_{k+1}$ corresponding to time instance k based on the ground truth value $\mathbf{x}_{k+1}$ and its preceding ground truth values $\mathbf{X}_k$ and $\mathbf{y}_k$ from the first set of training data $X_{train}$. In this manner the backward model g is trained using the first set of training data (or a subset thereof) into trained backward model g*. These trainings of models f and g into trained models f* and g* are illustrated in the upper part of Fig. 1.

Once the training of f into trained model f* and the training of g into trained model g* have been completed, these two trained models are used to generate a second set of training data, which will then be used to "further" train the model f*, which has been trained using the first set of training data $X_{train}$ . This will be explained in connection with the lower half of Fig. 1.

For the generation of the second set of training data there is used another set of data comprising ground truth values, which can be called "generative data" or "validation data" $X_{val}$ or $X_{gen}$ as illustrated in Fig. 1. This data $X_{gen}$ , like the first set of training data, also comprises 1... K pairs of values $\mathbf{X}_k$ and $\mathbf{y}_k$ which correspond respectively to k instances of time, with k preceding k+1 in time and with a pair of values $\mathbf{X}_k$ and $\mathbf{y}_k$ at time k being ground truth values of $\mathbf{X}_k$ and $\mathbf{y}_k$

The data sets $X_{train}$ and $X_{gen}$ thereby preferably are disjunct, i.e. they do not have any members in common. In other words, the data set $X_{gen}$ or its members preferably have neither been uses to train model f nor model g.

Using the generative data set $X_{gen}$ there is then generated the second training data set as follows.

The input feature value $\mathbf{X}_k$ at time instance k and its corresponding predicted target variable $\hat{\mathbf{y}}_k$ which has been predicted by the trained (forward) model f* are inputted into the backward model g* to predict the subsequent input feature value value $\hat{\mathbf{x}}_{k+1}$ . The predicted value $\hat{\mathbf{x}}_{k+1}$ which is predicted using the backward model g* and its corresponding ground truth value $\mathbf{y}_k$ from the generative data set $X_{gen}$ then form a pair of values of the second set of training data for the time instance k. In this manner, values $\mathbf{X}_k$ and $\mathbf{y}_k$ for 1....K-1 are generate as members of the second set of training data.

This second set of training data, which may be called or regarded as some kind of "replay data", is then used to further train the model f*. In this manner, the resiliency of the model f* against prediction errors can be improved, and its capability to adapt to changing environments can be enhanced.

In the above manner the model f* can be trained by two training data sets, the second training in some sense resembling a classical replay approach, however, instead of already used training data comprising ground truth values there is used a second set of training data as a "replay buffer", which has been generated by a forward-backward model, which incorporates both, a prediction of the target variable using a forward model f* and a prediction of the input feature value using the backward model g*.

The forward model f* has been trained using a first set of training data $(y_k, x_k)$ corresponding to k =1... N time points to find the trained machine learning model f*, and the backward model g* has been trained based on at least a part of the first set of training data $(y_k, x_k)$ to predict an input feature value $x_{k+1}$ based on its preceding input feature value $x_k$ and the corresponding ground truth target variable $y_k$:

Using the trained backward model g* there is then generated the second set of training data as a "replay buffer", which comprises the future input feature values $\hat{x}_{k+1}$ predicted by the backward machine learning model g* and the corresponding ground truth variable $\mathbf{y}_{k+1}$. This replay buffer is then used to further train the model f* to improve its resilience against prediction errors and its performance. The thus trained model that superior performance under conditions of distribution shift, real-world noise, and model errors.

It further improves its resilience against a phenomenon known as "catastrophic forgetting":

Especially when a model learns a new task during multi-task continual learning, it might forget the old tasks. To deal with the forgetting problem, different strategies are used, one of them being so-called replay strategies. Replay strategies have been mostly successful in dealing with forgetting by storing the training samples from the older tasks, which are then replayed along with the samples belonging to the new task.

According to one embodiment, the model is trained to control a bionic limb, which has motors or actuators embedded, which are controlled by the trained model. The bionic limb in one embodiment may be a lower limb, and the model is used to control the limb such that it emulates the natural gait. In such a setting of prosthetic gait generation using learning-based models, the stored training samples from old tasks can become obsolete. For example, if the predictions from the gait-prediction model (the so-called forward model) are used to generate a prosthetic gait, the prediction errors at the current time point influences the input patterns in the next time point.

To incorporate this sequential effect of model errors, we there is trained a backward model, which predicts the input pattern at the next time point given the input pattern and prediction from the original model at the current time point. For the original model to be robust to the prediction errors affecting the input patterns, it should be trained to map these deviated input patterns to desired output. This introduces an inductive bias on autocorrecting model errors, which makes it robust to inputs being affected due to prediction errors.

In one embodiment, the backward model is used as a generative model to generate new input patterns to update the original model with error feedback. It learns a functional mapping g* from the input $x_k$ and target $y_k$ at time-point k to the input $\hat{x}_{k+1}$ at time-point k + 1, such that

$$\mathbf{g}^* = arg\, min\, \left(\textstyle\sum_{k=1}^{K-1}(x_{k+1} - g(x_k, y_k))^2\right)$$

Based thereon then a second training data set, which may be called replay buffer, is generated using a separate held-out generative data set of validation data set, which is neither used in training nor testing the original model f. The replay buffer in one embodiment consists of the backward-model predicted future inputs and the corresponding ground truth values of the target gait variable $\{(g^*(x_k, y_k), y_{k+1}), k = 1..K - 1\}$, where K is the number of samples in the held-out generative data set. The thus generated replay buffer is then used to further train the model f*.

It should be noted that in other embodiments the described approach may as well be used to control other physical systems than a bionic limb, which have motors or actuators controlled by model f*.

According to a further embodiment, a multi-task adaptive learning approach is used. In multi-task adaptive learning, the model learns multiple tasks as well as refines its knowledge of each task with time. Multi-task incremental learning is used e.g. in assistive robotics which requires learning new tasks on the go without forgetting the old ones. The current continual learning strategies rely mostly on replaying the samples from older tasks to preserve the knowledge of those tasks while learning new ones. By using a replay approach based on the generated second training data set as described in previous embodiments, a multi-task incremental learning in one embodiment is implemented for a for time series regression problem in the context of training e.g. a multi-task adaptive gait-prediction model. In this approach, the model can incrementally learn new tasks and continually adapt to the temporal variations in the old tasks.

Assume that in one embodiment

$$\mathbf{X}_t \in \mathbb{R}^{N_t \times d}$$

and

$$y_t \in \mathbb{R}^{N_t}$$

be the input features and target variables respectively for task $t \in T$ where

$$T \subset \mathcal{T}$$

is the set of all locomotion tasks which are encountered from a space of all possible locomotion tasks $\mathcal{T}$, Nt is the number of samples belonging to task t, and d is the dimensionality of inputs.

In such a scenario, the gait adaptation for multiple locomotion tasks can be formulated as a multi-task continual learning problem with error feedback that can directly predict the prosthesis commands (position or velocity, e.g. angular position) while adapting to new tasks and potential model errors. To adapt to new tasks while preserving the learning of older tasks, in one embodiment there is used a combination of task-incremental learning and multi-head architecture with a shared backbone Θs, and task-specific layers Θt. When a new task t' is encountered, a new task specific layer **Οt',** is added and the model is retrained to adapt to the new task.

To prevent forgetting the old tasks and adapt to potential model errors, the model is trained using samples of the old tasks generated using a backward model g that maps the input patterns and the target variables at the current time-point $(x_k, y_k) \in (X_t, y_t)$, $k = 1..N_t - 1$ to the subsequent input patterns $x_{k+1} \in X_t$ for each of the old tasks $t \in T$. Based thereon a gait prediction model $f : \mathbb{R}^d \to \mathbb{R}$ is determined such that

$$\Theta_s^*, \Theta_t^* = \underset{\{\Theta_s, \Theta_t, t \in T\}}{\arg\min} \left( \left\| \mathbf{y}_{t'} - f(\mathbf{X}_{t'}; \Theta_s, \Theta_{t'}) \right\|_2 + \sum_{t \in T} \left\| \mathbf{y}_t - f(\hat{\mathbf{X}}_t; \Theta_s, \Theta_t) \right\|_2 \right), t \in T \cup t'$$

where

$$\hat{X}t = g_t{}^*(\mathbf{X}_t, f(\mathbf{X}_t)).$$

The first term on the right-hand side of the equation updates the task-specific layers of the newly encountered task t' using task-specific training data and the second term updates the task-specific layers of previously learned tasks $t \in T$ using samples generated by the backward model gt. Both terms also update the parameters of the shared backbone.

According to one embodiment an artificial neural network, preferably a temporal convolutional network TCN, is used for the models f and/or g. In one embodiment a TCN is used for the shared layer. The overall model may be a single head prediction model, consists of a TCN based shared module and a single prediction head that is shared by all the tasks. During training, the model learns a unified relation applicable to all the tasks. In alternative embodiment, a multi-head prediction model is used, which has a TCN-based shared layer and on prediction head per locomotion task on top of the TCN. During training, the shared layer is updated for input samples belonging to any task, whereas the task specific modules are updated using only those samples from the given task.

In a concrete implementation, according to one embodiment a shared backbone and task-specific prediction heads has been implemented, that allows sharing and transfer of information between tasks while allowing to differentiate between them and preserve learning of successive tasks. For the shared backbone, a TCN model based on "Shaojie Bai, J. Zico Kolter, and Vladlen Koltun. An empirical evaluation of generic convolutional and recurrent networks for sequence modeling.arXiv: 1803.01271, 2018" available at https://github.com/locuslab/TCN/ has been used. Prediction heads are placed after the final temporal block. For single-head models, a single prediction head is used and for multi-head models, one prediction head per task is used. Each prediction head (task-specific layers in case of a multi-head model) is a fully connected network. For training the models, we use a stochastic gradient descent optimizer with learning rate of 0.001 and momentum of 0.9.

The implementation has been evaluated by comparing it with existing models, which has shown that in particular the further training by the second training data set, the forward-backward replay buffer, significantly improves the performance of the model compared to existing approaches when being applied to gait prediction.

It should be noted that the described approach can be applied for a manifold of scenarios, with gait prediction, bionic limb control, and autonomous driving only being some of the possible specific examples. The described training method for a model and the resulting trained model may be used to control any machine or physical apparatus, where sensor data are obtained and are used to determine one or more control parameters in order to control the system by controlling its actuators or motors in real time based on the obtained sensor input data.

For example, in embodiments according to the invention the control parameter Y may be a control value for controlling a machine or apparatus, for example by controlling an engine, a motor, a steering mechanism, or an actuator of the machine or apparatus. The machine or apparatus may for example be any machine for manufacturing, driving, flying, or moving a physical entity. This may e.g. include such machines or apparatuses as:

a car;
a plane;
a vehicle;
a robot;
a machine used in a manufacturing plant.

Thereby the model f can be applied to control some real-world machines or apparatuses in a way, which is more resilient against prediction errors of the prediction model.

According to a further embodiment, the controlled machine or apparatus may also be a virtual machine or apparatus, which is simulated in a virtual scenario, and which is controlled by said model f.

The implementation of the controlled machine or apparatus as a simulated machine or apparatus enables the application, development, testing and improvement of the model even without experiments in the real-world but rather based on simulation scenarios. This can become more and more important, as digital twins are used for development, monitoring and implementation of real world scenarios such as machines, apparatuses, and even manufacturing plants.

According to one embodiment, there is also provided an apparatus, which is controlled by a computer-implemented model according to one of the embodiments of the invention.

**Claims**

1. A method for training a computer-implemented machine-learning model $f$ for controlling a system by control parameter $\mathbf{y}$, wherein said model has been trained to predict a target variable $\mathbf{y} = f(x)$ based on an input feature value $\mathbf{X}$, where $\mathbf{X}$ is an input value feature value obtained by one or more sensors and used to generate by said model said target variable y as control parameter to thereby control said system,

   wherein said model $f$ has been trained using a first set of training data $(\mathbf{y}_k, \mathbf{x}_k)$ corresponding to k =1... N time points to find the trained machine learning model $f^*$
   wherein a backward model g* has been trained based on at least a part of said first set of training data $(\mathbf{y}_k, \mathbf{x}_k)$ to predict an input feature value $\hat{x}_{k+1}$ based on its preceding input feature value $\mathbf{x}_k$ and the corresponding target variable $\mathbf{y}_k$, said method comprising:

       using said backward model g* to generate second set of training data, which comprises the future input feature values $\hat{x}_{k+1}$ predicted by the backward machine learning model g* and the corresponding ground truth variable $\mathbf{y}_{k+1}$,
       training said model f, which has been trained using said first set of training data, using said replay buffer as said second set of training data.

2. The method of claim 1, wherein
   said second set of training data comprises pairs of values comprising a future input feature value $\hat{x}_{k+1}$ , which is predicted by the trained backward model g* as $g^*(x_k, f(x_k))$ with $x_k$ being a ground truth input feature value from the first set of training data and $f(x_k)$ being its corresponding predicted target variable predicted by model f, and with $\mathbf{y}_{k+1}$ being the ground truth value from the first set of training data corresponding to the predicted input feature value $x_{k+1}$.

3. The method of claim 1 or 2, wherein the model g* has been trained using said first set of training data or a subset thereof, the first set of training data comprising $(\mathbf{y}_k, \mathbf{x}_k)$ for k=1... N, with N being the number of samples of the first set of training data.

4. The method of one of claims 1 to 3, wherein
   the pairs of values of the second set of training data correspond to the ground truth variable $\mathbf{y}_{k+1}$ of the first set of training data and the value $\hat{x}_{k+1}$ predicted by the backward model g* as $g^*(x_k, f(x_k))$ for all k = 1 to K-1, where K is the number of samples or a subset of samples of a separate set of generative data $(\mathbf{y}_k, \mathbf{x}_k)$ comprising ground truth values, which have not been used for training the models f or g*.

5. The method of one of claims 1 to 4, wherein
   said method comprises further training said trained model by said second set of training data generated using said trained backward machine learning model g* which has been trained using at least a part of said training data to learn a functional mapping g* to predict a future value $\hat{x}_{k+1}$ based on a temporally preceding feature value $\mathbf{x}_k$ and its corresponding target value $\mathbf{y}_k$ so that $\mathbf{x}_{k+1} = g(\mathbf{x}_k, \mathbf{y}_k)$ by finding the trained backward model as

$$\mathbf{g}^* = arg\ min\ \left(\sum_{k=1}^{K-1}(x_{k+1} - g(x_k, y_k))^2\right)$$

wherein said trained backward model g* is used to generate said second set of training data consisting of the future input feature values $\hat{x}_{k+1}$ predicted by the trained backward machine learning model $g^*(x_k, y_k)$ and the corresponding

ground truth variable $y_{k+1}$ from the set of generative data.

6. The method of one of claims 1 to 5, wherein

said model f is a neural network $f(\boldsymbol{X};\Theta)$ , preferably a temporal convolutional network TCN, with $\boldsymbol{X}$ being the input feature value and $\Theta$ being the trainable parameters of the one or more layers of the neural network; wherein said model has been trained using a first set of training data $(\boldsymbol{y}_k, \boldsymbol{x}_k)$ corresponding to k =1...M time points to find the trained neural network having the trained parameters $\Theta^*$ of the at least one layer of the neural network as

$$\Theta^* = \arg\min (\| \, y - f(\boldsymbol{X};\Theta \, \|_2)$$

wherein said second training set is used to further train said model by finding improved trained parameters of the at least one layer of the neural network as

$$\Theta^* = \arg\min (\| \, y - f(\hat{X};\Theta \, \|_2)$$

where

$$\hat{X} = g^*(\boldsymbol{X}, f(\boldsymbol{X})).$$

7. The method of any one of claims 1 to 6, wherein

said model f has at least two layers with corresponding training parameters, a shared layer $\Theta_s$ and one or more task specific layers $\Theta_t$ , where when a new task t' is to be learned, a new task specific layer $\Theta_{t'}$ is added and the model f is retrained to adapt to the new task t' to thereby obtain the trained model f having the layers $\Theta_s$ and $\Theta_t$ as

$$\Theta_s^*, \Theta_t^* = \underset{\{\Theta_s, \Theta_t, t \in T\}}{\arg\min} \left( \left\| \mathbf{y}_{t'} - f\left(\mathbf{X}_{t'}; \Theta_s, \Theta_{t'}\right) \right\|_2 + \right.$$

$$\left. \sum_{t \in T} \left\| \mathbf{y}_t - f(\hat{\mathbf{X}}_t; \Theta_s, \Theta_t) \right\|_2 \right), t \in T \cup t'$$

where

$$\hat{X}t = g_t^*(\mathbf{X}_t, f(\mathbf{X}_t)).$$

8. The method of one of the preceding claims, wherein

said control parameter Y is a control value for controlling a machine or apparatus, preferably by controlling an engine, a motor, a steering mechanism, or an actuator of said machine or apparatus, and wherein said said system comprises said machine or apparatus, preferably being a machine or apparatus for manufacturing, driving, flying, or moving a physical entity, more preferably being one of the following:

a car;
a plane;
a vehicle;
a robot;
a machine used in a manucturing plant.

9. The method of one of claims 1 to 8, wherein

said controlled machine or aparatus is a virtual machine or apparatus, which is simulated in a virtual scenario, and which is controlled by said model f.

10. The method of one of the preceding claims, wherein the input feature value $\boldsymbol{X}$ is based on sensor data, wherein the sensor data preferably comprises one or more of the following:

position sensor data;
velocity sensor data;
pressure sensor data;
voltage sensor data;
current sensor data;
magnetic field sensor data;
temperature sensor data;
acceleration sensor data;
wearable sensor data.

11. The method of one of the preceding claims, wherein

said control parameter **Y** is a control value for controlling one or more motors or actuators of a bionic limb, which is controlled by said model to enable a patient to emulate the motion of a human limb, and wherein preferably the control parameter **Y** comprises one or more of an angle, a speed, an acceleration, or a force for controlling one or more actuators or motors of said bionic limb.

12. The method of one of claims 1 to 9, wherein

said control parameter **Y** is a control value for controlling one or more motors or actuators of a car, preferably one or more of a steering wheel angle, a deceleration value, or an acceleration value, and wherein
the input feature value **X** is based on sensor data, preferably one or more of camera-based sensor data, LIDAR sensor data, or radar sensor data.

13. A computer-implemented machine-learning model *f* for controlling a system by control parameter **y,** wherein said model has been trained using a method according to one of claims 1 to 12.

14. Use of a computer-implemented machine-learning model *f* for controlling a system by control parameter **y,** wherein said model has been trained using a method according to one of claims 1 to 12.

15. A computer program, preferably recorded on a computer redable medium, comprising program executable code, which when being executed by a computer enables said computer to perform a method according to one of claims 1 to 12.

**Fig. 1**

Europäisches
Patentamt

European
Patent Office

Office européen
des brevets

**EUROPEAN SEARCH REPORT**

Application Number

EP 23 16 9568

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WENDONG ZHANG ET AL: "Predictive Experience Replay for Continual Visual Control and Forecasting", ARXIV.ORG, CORNELL UNIVERSITY LIBRARY, 201 OLIN LIBRARY CORNELL UNIVERSITY ITHACA, NY 14853, 12 March 2023 (2023-03-12), XP091459108, | 1-10, 13-15 | INV. G06N3/045 G06N3/0895 A61F2/48 A61F2/68 G06N3/0464 G06N3/096 |
| Y | * the whole document * | 11,12 | |
| A | Anonymous: "Imitation Learning", , 22 February 2021 (2021-02-22), pages 1-14, XP93088298, Retrieved from the Internet: URL:https://web.stanford.edu/class/cs237b/pdfs/lecture/lecture_10111213.pdf [retrieved on 2023-10-04] * Section 10.2 * | 6,7 | |
| A | WALLINGFORD MATTHEW ET AL: "Task Adaptive Parameter Sharing for Multi-Task Learning", 2022 IEEE/CVF CONFERENCE ON COMPUTER VISION AND PATTERN RECOGNITION (CVPR), IEEE, 18 June 2022 (2022-06-18), pages 7551-7560, XP034193621, DOI: 10.1109/CVPR52688.2022.00741 [retrieved on 2022-09-27] * abstract * * figure 1 * | 7 | TECHNICAL FIELDS SEARCHED (IPC) G06N A61F |

-/--

The present search report has been drawn up for all claims

| Place of search | Date of completion of the search | Examiner |
|---|---|---|
| The Hague | 6 October 2023 | Mariani, Giovanni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt
European
Patent Office
Office européen
des brevets

# EUROPEAN SEARCH REPORT

Application Number

EP 23 16 9568

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SHARMITA DEY ET AL: "A hybrid approach for dynamically training a torque prediction model for devising a human-machine interface control strategy", ARXIV.ORG, CORNELL UNIVERSITY LIBRARY, 201 OLIN LIBRARY CORNELL UNIVERSITY ITHACA, NY 14853, 6 October 2021 (2021-10-06), XP091071998, * abstract * * figure 1 * | 11 | |
| Y | MAYANK BANSAL ET AL: "ChauffeurNet: Learning to Drive by Imitating the Best and Synthesizing the Worst", ARXIV.ORG, CORNELL UNIVERSITY LIBRARY, 201 OLIN LIBRARY CORNELL UNIVERSITY ITHACA, NY 14853, 7 December 2018 (2018-12-07), XP080990446, * abstract * * Section 1 * | 12 | |

TECHNICAL FIELDS
SEARCHED (IPC)

The present search report has been drawn up for all claims

| Place of search | Date of completion of the search | Examiner |
|---|---|---|
| The Hague | 6 October 2023 | Mariani, Giovanni |

EPO FORM 1503 03.82 (P04C01)